# EUROPÄISCHE PATENTANMELDUNG

(11) **EP 3 181 129 A1**
(43) Veröffentlichungstag der Anmeldung: **21.06.2017**
(21) Anmeldenummer: 16153198.3
(22) Anmeldetag: 28.01.2016
(51) Int. Cl.: A61K 31/07, A61K 31/19, A61K 31/355, A61P 15/00

(54) **ZUSAMMENSETZUNG ZUR BEHANDLUNG VON PRÄMENSTRUELLEM SYNDROM**

(30) Priorität: 15.12.2015 DE 202015106826 U
(71) Anmelder: Braun, Wolfgang, 86152 Augsburg (DE)
(72) Erfinder: Braun, Wolfgang, 86152 Augsburg (DE)
(74) Vertreter: Peter, Julian

(57) **Zusammenfassung**

Die vorliegende Erfindung betrifft Zusammensetzungen zur Linderung und/oder Behandlung von Symptomen, welche mit dem prämenstruellen Syndrom (PMS) zusammenhängen. Ferner betrifft die vorliegende Erfindung die Verwendung der erfindungsgemäßen Zusammensetzungen für die Herstellung einer Dosierungsform zur Behandlung von physischen und/oder körperlichen Symptomen einer PMS Patienten durch orale Verabreichung.

## Beschreibung

### TECHNISCHES GEBIET

Die vorliegende Erfindung betrifft eine pharmazeutische Zusammensetzung umfassend Oleinsäure, Gamma-Linolensäure, Linolsäure und mindestens ein Vitamin. Zusätzlich betrifft die vorliegende Erfindung die Verwendung der erfindungsgemäßen Zusammensetzung für die Herstellung einer Dosierungsform zur Behandlung von physischen Symptomen einer PMS Patienten durch orale Verabreichung, wobei die Dosierungsform in einem Dosierungsschema verabreicht wird, in dem die Erstverabreichung 14 Tage vor der Menstruation 2 bis 6 Mal täglich durchgeführt wird.

### HINTERGRUND DER ERFINDUNG

Das prämenstruelle Syndrom (PMS) ist ein sehr häufiges Phänomen. Bei Frauen im gebärfähigen Alter treten die PMS-Symptome etwa zwei Wochen bis drei Tage vor der Menstruationsblutung auf. Sind PMS-Symptome nur leicht ausgeprägt, muss nicht unbedingt eine Behandlung erfolgen. In schweren Fällen können die Symptome jedoch den Alltagsleben, das Familien- und Berufsleben beeinträchtigen.

PMS-Symptome können in körperliche und psychische Symptome unterteilt werden. Bei den meisten Frauen zeigen sich Symptome beider Art, bei einigen treten aber auch nur körperliche oder nur psychische Symptome auf.

Ein großes Problem bei PMS sind Stimmungsschwankungen. Viele Frauen geraten von einem Moment zum anderen vollkommen ohne Grund in traurige, ängstliche oder sogar depressive Stimmungen. Sind diese Symptome sehr stark ausgeprägt, sollte eine psychiatrische Beratung und eventuell eine begleitende Psychotherapie in Erwägung gezogen werden.

Vorschläge zur Behandlung von PMS Symptomen umfassen überwiegend die Verabreichung von Pharmazeutika, wie Vitaminergänzungsmittel und Eierstockhormone. Diese Präparate zeigen jedoch einen begrenzten Erfolg. Derzeit gibt es kein Mittel zur Behandlung der Störungen der Gemütslage und des Appetites, welche gewöhnlich wiederholt durch eine große Anzahl von Frauen erfahren werden. Eine solche Behandlung wäre von großem Vorteil.

Die Aufgabe der vorliegenden Erfindung besteht in der Bereitstellung einer pharmazeutischen Zusammensetzung zur Behandlung physischer Symptomen einer PMS Patienten. Eine weitere Aufgabe der vorliegenden Erfindung besteht in der Bereitstellung einer verbesserten Therapie für die Patienten, die unter PMS leiden. Eine dritte Aufgabe der vorliegenden Erfindung besteht in der Bereitstellung einer Zusammensetzung, die zusätzlich die körperliche Leistung der Frauen, die unter PMS leiden, steigert.

### ZUSAMMENFASSUNG DER ERFINDUNG

Diese Aufgabe wird durch die vorliegende Erfindung gelöst, die eine pharmazeutische Zusammensetzung bereitstellt, welche Oleinsäure, Gamma-Linolensäure, Linolsäure und mindestens ein Vitamin umfasst. Die erfindungsgemäße pharmazeutische Zusammensetzung ist zur Behandlung physischer und / oder körperlicher Symptomen einer PMS Patienten vorgesehen.

Die erfindungsgemäße pharmazeutische Zusammensetzung umfasst mindestens ein Vitamin ausgewählt aus der Gruppe bestehend aus Retinol, Thiamin, Riboflavin, Niacin, Pantothensäure, Pyridoxin, Biotin, Folsäure, Cobalamin, Ascorbinsäure, Calcitriol, Phyllochinon, α-Tocopherol, oder Äquivalente davon.

In einer Ausführungsform umfasst die erfindungsgemäße pharmazeutische Zusammensetzung zwischen 1 mg und 25 mg Retinol. In einer weiteren Ausführungsform umfasst die erfindungsgemäße pharmazeutische Zusammensetzung 20 mg Retinol.

In einer Ausführungsform umfasst die erfindungsgemäße pharmazeutische Zusammensetzung 400 I.E. α-Tocopherol.

In einer weiteren Ausführungsform umfasst die erfindungsgemäße pharmazeutische Zusammensetzung zwischen 25 mg und 200 mg, vorzugs-weise zwischen 125 mg und 175 mg Oleinsäure.

In einer Ausführungsform umfasst die erfindungsgemäße pharmazeutische Zusammensetzung zwischen 50 mg und 400 mg Gamma-Linolensäure. In einer weiteren Ausführungsform umfasst die erfindungsgemäße pharmazeutische Zusammensetzung zwischen 100 mg und 200 mg Gamma-Linolensäure.

Die erfindungsgemäße pharmazeutische Zusammensetzung umfasst zwischen 200 mg und 450 mg Linolsäure, vorzugsweise zwischen 300 mg und 345 mg Linolsäure.

Die erfindungsgemäße pharmazeutische Zusammensetzung kann auch zusätzlich Magnesium und/oder eine Calciumverbindung umfassen. In dem Fall wird eine Calciumverbindung aus einer Gruppe bestehend aus Calciumcarbonat, Calciumcitrat und deren Kombinationen ausgewählt.

Die erfindungsgemäße pharmazeutische Zusammensetzung wird über eine orale Route verabreicht.

Ferner betrifft die vorliegenden Erfindung die Verwendung der erfindungsgemäßen Zusammensetzung für die Herstellung einer Dosierungsform zur Behandlung von physischen Symptomen einer PMS Patienten durch orale Verabreichung, wobei die Dosierungsform in einem Dosierungsschema verabreicht wird, in dem die Erstverabreichung 14 Tage vor der Menstruation 2 bis 6 Mal täglich durchgeführt wird. Durch die Verabreichung der erfindungsgemäßen Zusammensetzungen nach dem hier definierten Dosierungsschema werden auch die körperlichen Symptome des PMS behandelt.

### DETAILLIERTE BESCHREIBUNG DER ERFINDUNG

Die Zusammensetzungen, die Gegenstand der vorliegenden Erfindung darstellen, weisen den Vorteil auf, dass sie gut verträglich und individuell dosierbar sind. Die erfindungsgemäßen Zusammensetzungen enthalten eine neuartige Kombination aus Oleinsäure, Gamma-Linolensäure, Linolsäure mit verschiedenen Vitaminen und / oder Mineralstoffen.

Die vorliegende Erfindung betrifft eine pharmazeutische Zusammensetzung, welche Oleinsäure, Gamma-Linolensäure, Linolsäure und mindestens ein Vitamin umfasst. Die erfindungsgemäße pharmazeutische Zusammensetzung ist zur Behandlung physischer und/oder körperlicher Symptomen einer PMS Patienten vorgesehen. Insbesondere Stimmungsschwankungen werden durch die Verabreichung erfindungsgemäßen Zusammensetzungen mit Erfolg behandelt.

Ohne auf die Theorie beschränkt zu sein, stimulieren die erfindungsgemäßen Zusammensetzungen einen oder mehrere biologische Routen. Beispielsweise durch die Zugabe von Ferrum phosphoricum wird eine erhöhte Sauerstoffaufnahme angeregt. Gleichzeitig wird eine optimale Nahrungsergänzung bei Frauen, die unter PMS leiden, erzielt.

Die Behandlung erfolgt durch die Verabreichung der erfindungsgemäßen Zusammensetzungen 2 bis 6 Mal täglich, je nach Bedarf der Patientin. Die vorliegenden Zusammensetzungen können in Form von Gelatinekapseln verabreicht werden. Bei schwerwiegenden depressiven Zuständen können die Patienten bis zu 12 Gelatinekapseln am Tag zu sich nehmen. Bei leichten Stimmungsschwankungen können die Patienten 2 bis 4 Gelatinekapseln einnehmen.

Die vorliegende Erfindung betrifft auch ein Dosierungsschema, welches individuell nach den Bedürfnissen der Patienten angepasst wird. Nach dem erfindungsgemäßen Dosierungsschema erfolgt die Erstverabreichung der vorliegenden Zusammensetzungen 14 Tage vor der Menstruation 2 bis 6 Mal täglich. In einer Ausführungsform bekommen die Patienten 14 Tage vor der Menstruation 2 Gelatinekapseln am Tag, jeweils Morgens und Abends vor den Mahlzeiten. In einer anderen Ausführungsform bekommen die Patienten 14 Tage vor der Menstruation 6 Gelatinekapseln am Tag, verteilt auf drei Mahlzeiten, jeweils zwei Gelatinekapseln vor den Mahlzeiten. In einer weiteren Ausführungsform bekommen die Patienten 14 Tage vor der Menstruation 12 Gelatinekapseln am Tag, verteilt auf drei Mahlzeiten, jeweils 4 Gelatinekapseln vor den Mahlzeiten. In einer Ausführungsform erfolgt die Einnahme vor der Mahlzeit. In einer weiteren Ausführungsform erfolgt die Einnahme nach der Mahlzeit.

Das Dosierungsschema hat eine Dauer von 4 Wochen, wobei die Erstverabreichung 14 Tage vor der Menstruation durchgeführt wird. In einer Ausführungsform hat das Dosierungsschema eine Dauer von 3 Wochen, wobei die Erstverabreichung 14 Tage vor der Menstruation durchgeführt wird.

Die erfindungsgemäße pharmazeutische Zusammensetzung umfasst mindestens ein Vitamin. Bevorzugte Vitamine sind ausgewählt aus der Gruppe bestehend aus Retinol, Vitamin-A-Propionat, Vitamin-A-Palmitat, Vitamin-A-Acetat, Thiamin, Riboflavin, Niacin, Pantothensäure, Pyridoxin, Biotin, Folsäure, Cobalamin (Vitamin B₁₂), Ascorbinsäure, Calcitriol, Phyllochinon, α-Tocopherol, Tocopherol-E-Acetat, DL-α-Tocopherol, Tocopherolhydrogensuccinat, Derivate oder Äquivalente davon.

Wenn die erfindungsgemäße Zusammensetzung Retinol enthält, liegt die Menge an Retinol bevorzugt von 1 mg bis 25 mg vor. Besonders bevorzugt liegt die Menge an Retinol von 20 mg bis 25 mg vor.

In einer Ausführungsform enthält die Zusammensetzung 20 mg Retinol.

In einer Ausführungsform umfasst die erfindungsgemäße pharmazeutische Zusammensetzung 400 I.E. α-Tocopherol. Die Begriffe "Vitamin E" und "α-Tocopherol" sind gleichbedeutend. Wenn α-Tocopherol (Vitamin E) in der erfindungsgemäßen Zusammensetzung vorhanden ist, liegt es bevorzugt in einer Menge von 20 mg bis 500 mg vor. Besonders bevorzugt liegt das Vitamin E (α-Tocopherol) in einer Menge von 25 mg bis 450 mg vor.

In einer weiteren Ausführungsform umfasst die erfindungsgemäße Zusammensetzung eine Kombination aus 20 mg Retinol und 400 I.E. α-Tocopherol.

In einer Ausführungsform umfasst die erfindungsgemäße Zusammensetzung folgende Komponente:
zwischen 125 mg und 175 mg Oleinsäure;
zwischen 100 mg und 200 mg Gamma-Linolensäure;
zwischen 300 mg und 345 mg Linolsäure;
20 mg Retinol und 400 I.E. α-Tocopherol.

In einer Ausführungsform umfasst die erfindungsgemäße pharmazeutische Zusammensetzung zwischen 25 mg und 200 mg, vorzugsweise zwischen 125 mg und 175 mg Oleinsäure.

In einer weiteren Ausführungsform umfasst die erfindungsgemäße pharmazeutische Zusammensetzung zwischen 50 mg und 400 mg, vorzugs-weise zwischen 100 mg und 200 mg Gamma-Linolensäure.

Die erfindungsgemäße pharmazeutische Zusammensetzung umfasst zwischen 200 mg und 450 mg Linolsäure, vorzugsweise zwischen 300 mg und 345 mg Linolsäure.

Die erfindungsgemäße pharmazeutische Zusammensetzung kann zusätzlich Magnesium und/oder eine Calciumverbindung umfassen. In dem Fall wird eine Calciumverbindung aus einer Gruppe bestehend aus Calciumcarbonat, Calciumcitrat und deren Kombinationen ausgewählt. Die Menge an Calciumverbindungen ist von 200 mg bis 3000 mg, vorzugsweise von 600 mg bis 1000 mg. Die Menge an Magnesium ist von 25 mg bis 500 mg, vorzugsweise von 25 mg bis 350 mg.

In einer weiteren Ausführungsform umfasst die erfindungsgemäße Zusammensetzung eine Kombination aus 20 mg Retinol und 400 I.E. α-Tocopherol mit Magnesium.

In einer weiteren Ausführungsform umfasst die erfindungsgemäße Zusammensetzung eine Kombination aus 20 mg Retinol und 400 I.E. α-Tocopherol mit einer Calciumverbindung.

Die erfindungsgemäßen Zusammensetzungen können auch Kombinationen an weiteren Mineralstoffen umfassen. Beispiele sind Ferrum phosphoricum, Kalium phosphoricum und Magnesium phosphoricum. Ferrum phosphoricum sorgt für eine erhöhte Sauerstoffaufnahme. Kalium phosphoricum verstärkt die Nerven und Psyche. Magnesium phosphoricum unterstützt die Muskeln und Nerven.

Die Menge an Ferrum phosphoricum, Kalium phosphoricum und Magnesium phosphoricum beträgt jeweils von 200 mg bis 3000 mg, vorzugsweise von 600 mg bis 1000 mg.

In einer Ausführungsform umfasst die erfindungsgemäße Zusammensetzung folgende Komponente: Oleinsäure, Gamma-Linolensäure, Linolsäure, Retinol, α-Tocopherol, und mindestens ein Minerstoff ausgewählt aus der Gruppe bestehend aus Ferrum phosphoricum, Kalium phosphoricum und Magnesium phosphoricum.

Die erfindungsgemäße pharmazeutische Zusammensetzung wird über eine orale Route verabreicht. Der Erfindungsgegenstand umfasst jegliche denkbare Arzneiform, die die erfindungsgemäßen Zusammensetzungen enthält. Beispiele für derartige Arzneiformen umfassen ohne Einschränkung darauf Kautabletten, schnell lösliche Tabletten, Brausetabletten, rekonstituierbare Pulver, Lösungen, Suspensionen, Emulsionen, Nanosuspensionen, Nanoemulsionen, Tabletten, Mehrschichttabletten, Zweischichttabletten, Kapseln, Weichgelatinekapseln, Hartgelatinekapseln, Caplets, Lutschtabletten, Kau-Lutschtablette, Perlen, Pulver, Granulate, Pellets, Teilchen, Mikropartikel, Nanopartikel, dispergierbares Granulat, Stärkekapseln, Pulver zur Inhalation und deren Kombinationen.

In einer Ausführungsform wird die vorliegende Zusammensetzung in Hartgelatinekapseln oder in Weichgelatinekapseln abgefüllt.

Beispiele für pharmazeutisch inerte Trägerstoffe, die für die Zusammensetzung zum Füllen von Kapseln gemäß der Erfindung geeignet sind, sind fette Stoffe pflanzlichen Ursprungs, wie z. B. Kakao- und Karnaubawachs, pflanzliche Öle, wie z. B. Erdnußöl, Baumwollsamenöl, Maisöl, Olivenöl, Palmkernöl und Sojabohnenöl; hydrierte pflanzliche Öle; fette Stoffe tierischen Ursprungs, wie z. B. Spermacet, Bienenwachs und Lanolin und seine Derivate, Kohlenwasserstoffe; Zeresin; Mineralöl; Paraffinwachs; C12-18-Fettalkohole, wie z. B. Cetylalkohol und Stearylalkohol; C12-18-Fettsäuren, wie z. B. Laurinsäure, Myristinsäure, Palmitinsäure und Stearinsäure; Fettsäureester, wie z. B. Glycerylstearat, Isopropylmyristat und Äthyloleat; gemischte Ester, die feste halbsynthetische Glyceride sein können, wie z. B. Witepsol^{®}, Suppocire^{®}, Massa Esterinum^{®} und Massupol^{®}, oder flüssige halbsynthetische Glyceride, wie z. B. Miglyol 812^{®} und Labrafils^{®}; Amide oder alkoholische Amide von Fettsäuren, wie z. B. Comperlan KD^{®} und Ciramide^{®}; Metallstearate, wie z. B. eine Mischung der Di- und Tristearate von Aluminium; flüssige oder feste Silikone, wie z. B. Dimethylpolysiloxan; Macrogole wie z. B. die Carbowaxe^{®}, und Siliciumdioxidderivate, wie z. B. Bentonit, Veegum^{®}, Cab-O-Sil^{®} und Aerosil^{®}.

Die Zusammensetzungen zum Füllen von Kapseln gemäß der Erfindung kann man in Gelatinekapseln einfüllen, indem man eine Standardvorrichtung zum automatischen Kapselfüllen verwendet.

### BEISPIELE

Die folgenden erfindungsgemäßen Zusammensetzungen werden zur Verwendung / Verabreichung an Frauen, die unter PMS leiden eingesetzt:

### Beispiel 1:

| **Komponente** | **Dosis** | **Einheiten** |
|---|---|---|
| Oleinsäure | 175 | mg |
| Gamma-Linolensäure | 210 | mg |
| Linolsäure | 345 | mg |
| Retinol | 20 | mg |
| α-Tocopherol | 400 | I.E.* |

| | | |
|---|---|---|
| *I.E.: Internationale Einheit | | |

### Beispiel 2:

| **Komponente** | **Dosis** | **Einheiten** |
|---|---|---|
| Oleinsäure | 175 | mg |
| Gamma-Linolensäure | 210 | mg |
| Linolsäure | 345 | mg |
| Retinol | 20 | mg |
| α-Tocopherol | 400 | I.E.* |
| Ferrum phosphoricum | 250 | mg |

| | | |
|---|---|---|
| *I.E.: Internationale Einheit | | |

### Beispiel 3:

| **Komponente** | **Dosis** | **Einheiten** |
|---|---|---|
| Oleinsäure | 175 | mg |
| Gamma-Linolensäure | 210 | mg |
| Linolsäure | 345 | mg |
| Retinol | 20 | mg |
| α-Tocopherol | 400 | I.E.* |
| Ferrum phosphoricum | 125 | mg |
| Magnesium phosphoricum | 125 | mg |

| | | |
|---|---|---|
| *I.E.: Internationale Einheit | | |

### Beispiel 4:

| **Komponente** | **Dosis** | **Einheiten** |
|---|---|---|
| Oleinsäure | 175 | mg |
| Gamma-Linolensäure | 210 | mg |
| Linolsäure | 345 | mg |
| Retinol | 20 | mg |
| Kalium Phosphoricum | 25 | mg |
| Ferrum phosphoricum | 100 | mg |
| Magnesium phosphoricum | 125 | mg |

## Patentansprüche

1. Pharmazeutische Zusammensetzung umfassend Oleinsäure, Gamma-Linolensäure, Linolsäure und mindestens ein Vitamin.

2. Pharmazeutische Zusammensetzung nach Anspruch 1, wobei das mindestens ein Vitamin ausgewählt ist aus der Gruppe bestehend aus Retinol, Thiamin, Riboflavin, Niacin, Pantothensäure, Pyridoxin, Biotin, Folsäure, Cobalamin, Ascorbinsäure, Calcitriol, Phyllochinon, α-Tocopherol, Derivate oder Äquivalente davon.

3. Pharmazeutische Zusammensetzung nach Anspruch 1 oder 2, wobei die Zusammensetzung von 1 mg bis 25 mg Retinol umfasst.

4. Pharmazeutische Zusammensetzung nach einem der vorangehenden Ansprüche, wobei die Zusammensetzung 20 mg Retinol umfasst.

5. Pharmazeutische Zusammensetzung nach einem der vorangehenden Ansprüche, wobei die Zusammensetzung 400 I.E. α-Tocopherol umfasst.

6. Pharmazeutische Zusammensetzung nach einem der vorangehenden Ansprüche, wobei die Zusammensetzung von 25 mg bis 200 mg, bevorzugt von 125 mg bis 175 mg Oleinsäure umfasst.

7. Pharmazeutische Zusammensetzung nach einem der vorangehenden Ansprüche, wobei die Zusammensetzung von 50 mg bis 400 mg, bevorzugt von 100 mg bis 200 mg Gamma-Linolensäure umfasst.

8. Pharmazeutische Zusammensetzung nach einem der vorangehenden Ansprüche, wobei die Zusammensetzung von 200 mg bis 450 mg, bevorzugt von 300 mg bis 345 mg Linolsäure umfasst.

9. Pharmazeutische Zusammensetzung nach einem der vorangehenden Ansprüche, wobei die Zusammensetzung zusätzlich Magnesium und/oder eine Calciumverbindung umfasst.

10. Pharmazeutische Zusammensetzung nach Anspruch 9, wobei die Calciumverbindung ausgewählt ist aus einer Gruppe bestehend aus Calciumcarbonat, Calciumcitrat und deren Kombinationen.

11. Pharmazeutische Zusammensetzung nach einem der vorangehenden Ansprüche, wobei die Zusammensetzung zusätzlich Ferrum phosphoricum, Kalium phosphoricum und/oder Magnesium phosphoricum umfasst.

12. Pharmazeutische Zusammensetzung nach einem der vorangehenden Ansprüche, wobei die Zusammensetzung über eine orale Route verabreicht wird.

13. Pharmazeutische Zusammensetzung nach einem der vorangehenden Ansprüche, wobei die Zusammensetzung zur Behandlung physischer Symptomen einer PMS Patienten vorgesehen ist.

14. Verwendung der Zusammensetzung nach einem der vorangehenden Ansprüche für die Herstellung einer Dosierungsform zur Behandlung von physischen Symptomen einer PMS Patienten durch orale Verabreichung, wobei die Dosierungsform in einem Dosierungsschema verabreicht wird, in dem die Erstverabreichung 14 Tage vor der Menstruation 2 bis 6 Mal täglich durchgeführt wird.
